(19) Europäisches Patentamt
European Patent Office
Office européen des brevets

(11) **EP 4 471 705 A1**

(12) **EUROPEAN PATENT APPLICATION**
published in accordance with Art. 153(4) EPC

(43) Date of publication:
**04.12.2024 Bulletin 2024/49**

(21) Application number: **22930955.4**

(22) Date of filing: **11.03.2022**

(51) International Patent Classification (IPC):
**G06Q 50/02** *(2024.01)*     **A01G 7/00** *(2006.01)*

(52) Cooperative Patent Classification (CPC):
**A01G 7/00; G06Q 50/02**

(86) International application number:
**PCT/JP2022/011125**

(87) International publication number:
**WO 2023/170975 (14.09.2023 Gazette 2023/37)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Omron Corporation
Kyoto-shi, Kyoto 600-8530 (JP)**

(72) Inventors:
• **WANG, Ying
Kyoto-shi, Kyoto 600-8530 (JP)**
• **MIYAJI, Takaaki
Kyoto-shi, Kyoto 600-8530 (JP)**

(74) Representative: **Isarpatent
Patent- und Rechtsanwälte
Barth Hassa Peckmann & Partner mbB
Friedrichstraße 31
80801 München (DE)**

(54) **TRAINING METHOD, LEAF STATE IDENTIFICATION DEVICE, AND PROGRAM**

(57)     A learning method includes a weight determination step of determining a weight for a leaf included in a captured image and a first learning step of performing learning of a leaf detection model for detecting a leaf from the captured image based on the weight determined in the weight determination step such that a leaf having a large weight is more easily detected than a leaf having a small weight.

**FIG. 1A**

```
        ┌─────────┐
        │  START  │
        └────┬────┘
             │
             ▼
     ┌────────────────┐
     │   DETERMINE    │ ─── S101
     │ WEIGHT FOR LEAF│
     └───────┬────────┘
             │
             ▼
     ┌────────────────┐
     │   LEARN LEAF   │ ─── S102
     │ DETECTION MODEL│
     └───────┬────────┘
             │
             ▼
        ┌─────────┐
        │   END   │
        └─────────┘
```

**(Cont. next page)**

FIG. 1B

LEAF STATE IDENTIFICATION DEVICE

ACQUISITION UNIT

DETECTOR

LEAF DETECTION MODEL

IDENTIFICATION UNIT

LEAF STATE IDENTIFICATION MODEL

111

112

113

110

**Description**

TECHNICAL FIELD

The present invention relates to a technique for detecting a leaf and identifying a leaf state.

BACKGROUND ART

Since diseases and insect damages may greatly damage agricultural production, it is very important to discover the diseases and insect damages at an early stage and take measures. However, in a case of visually discovering the diseases and insect damages, it is difficult to perform early discovery and troublesome unless an agricultural expert (a person having specialized knowledge in agriculture).

Thus, a system that automatically discovers the diseases and insect damages has been proposed. Non-Patent Document 1 discloses a system that detects (extracts) a leaf from a captured image and identifies a state of the detected leaf.

CITATION LIST

NON-PATENT DOCUMENT

Non-Patent Document 1: Proceedings of the Graduate School of Science and Technology, Hosei University, vol. 58, pages 1 to 4, issued on March 31, 2017

SUMMARY OF INVENTION

PROBLEMS TO BE SOLVED BY INVENTION

However, in the technique disclosed in Non-Patent Document 1, in a case where a leaf (for example, a leaf that looks elongated, a leaf that looks small, a leaf that is partially hidden by another leaf, a blurred leaf that is out of focus, a dark leaf, or the like.) that is not suitable for identification of a leaf state (state of a leaf) is detected, an incorrect identification result is obtained for the leaf, and an overall identification accuracy decreases. Then, in a case where the overall identification accuracy is low, work (labor) such as confirmation of the identification result by the agricultural expert is required.

The present invention has been made in view of the above circumstances, and an object thereof is to provide a method for suitably detecting a leaf, and eventually performing a post-process such as identification of a leaf state with high accuracy.

MEANS FOR SOLVING THE PROBLEM

In order to achieve the above object, the present invention employs the following method.

A first aspect of the present invention provides a learning method including a weight determination step of determining a weight for a leaf included in a captured image; and a first learning step of performing learning of a leaf detection model for detecting a leaf from the captured image based on the weight determined in the weight determination step such that a leaf having a large weight is more easily detected than a leaf having a small weight.

According to the above-described method, a weight is determined for the leaf, and the learning of the leaf detection model is performed such that the leaf having the large weight is more easily detected than the leaf having the small weight. In this way, a leaf can be suitably detected, and eventually, post-process such as identification of the leaf state can be performed with high accuracy. For example, when a large weight is determined for a leaf suitable for the post-process and a small weight is determined (or no weight is determined) for a leaf not suitable for the post-process, the leaf suitable for the post-process is more easily detected than the leaf not suitable for the post-process.

In the weight determination step, a weight based on knowledge about agriculture may be determined. For example, in the weight determination step, a weight based on knowledge obtained from at least one of a visual line of an agricultural expert and experience regarding agriculture may be determined. In this way, the large weight can be determined for the leave suitable for the post-process, and the small weight can be determined (or no weight can be determined) for the leave not suitable for the post-process.

In the weight determination step, the weight of the leaf may be determined based on at least one of a shape, a size, and a position of the leaf. For example, a leaf that looks elongated by being viewed obliquely or partially hidden by another leaf or the like is likely to be not suitable for the post-process such that the leaf state cannot be identified with high accuracy.

Thus, in the weight determination step, a larger weight may be determined for the leaf as the shape of a bounding box of the leaf is closer to a square. A leaf that is undeveloped or partially hidden by another leaf or the like is likely to be not suitable for the post-process such that the leaf state cannot be identified with high accuracy. Thus, in the weight determination step, a larger weight may be determined for the leaf as the size of the leaf is larger. In addition, since the closer to the ground, the higher the humidity, mold disease is more likely to occur in the leaf closer to the ground than in the leaf farther from the ground. Thus, in the weight determination step, a larger weight may be determined for the leaf as the leaf is closer to the ground. Since young leaves (upper leaves) are more affected by insect pests, in the weight determination step, a larger weight may be determined for a leaf as the leaf is farther from the ground. The bounding box of the leaf is a rectangular frame surrounding the leaf, and may be, for example, a rectangular frame circumscribing the leaf.

The leaf detection model may be an inference model using Mask R-CNN or Faster R-CNN. In the first learning step, a value of a loss function may be reduced with a larger reduction amount as the weight is larger. In this way, an allowable range of the leaf is adjusted such that the allowable range based on the leaf having the large weight is wide and the allowable range based on the leaf having the small weight is narrow. As a result, the leaf having the large weight (leaf included in the allowable range based on the leaf having the large weight) is more easily detected than the leaf having the small weight (leaf included in the allowable range based on the leaf having the small weight).

A second learning step of performing learning of a leaf state identification model for identifying a state of a leaf by using a detection result of the leaf detection model learned in the first learning step may be further included. In this way, a leaf detection model that can suitably detect a leaf can be obtained, and the leaf state identification model that can identify a leaf with high accuracy can be obtained. The leaf state identification model may identify whether a leaf is affected by diseases and insect pests.

A second aspect of the present invention provides a leaf state identification device including an acquisition section configured to acquire a captured image, a detection section configured to detect a leaf from the captured image acquired by the acquisition section by using the leaf detection model learned by the learning method described above, and an identification section configured to identify a state of the leaf detected by the detection section by using a leaf state identification model for identifying a state of a leaf. According to this configuration, the leaf is detected using the leaf detection model learned by the learning method described above, and thus the leaf state can be identified with high accuracy.

Note that the present invention can be regarded as a learning device, a leaf state identification device, a learning system, or a leaf state identification system each including at least some of the above configurations or functions. In addition, the present invention can also be regarded as a learning method, a leaf state identification method, a control method of a learning system, or a control method of a leaf state identification system each including at least some of the above processes, or a program for causing a computer to execute these methods, or a computer-readable recording medium in which such a program is non-transiently recorded. The above-described components and processes can be combined with each other to configure the present invention as long as no technical contradiction occurs.

EFFECT OF INVENTION

According to the present invention, a leaf can be suitably detected, and eventually, post-process such as identification of the leaf state can be performed with high accuracy.

BRIEF DESCRIPTION OF THE DRAWINGS

[Fig. 1] Fig. 1A is a flowchart illustrating an example of a learning method to which the present invention is applied, and Fig. 1B is a block diagram illustrating a configuration example of a leaf state identification device to which the present invention is applied.
[Fig. 2] Fig. 2 is a block diagram illustrating a configuration example of a leaf state identification system according to the embodiment.
[Fig. 3] Fig. 3A is a flowchart illustrating an example of a process flow of a PC (leaf state identification device) in a learning phase, and Fig. 3B is a flowchart illustrating an example of a process flow of the PC in an inference phase after the learning phase.
[Fig. 4] Fig. 4A is a schematic view showing an example of a captured image for learning, and Fig. 4B and Fig. 4C are schematic views each showing an example of a bounding box and the like.
[Fig. 5] Fig. 5 is a schematic diagram illustrating an example of a leaf detection model using Mask R-CNN.
[Fig. 6] Fig. 6A shows a detection result (leaf detection result) before narrowing of a comparative example, and Fig. 6B shows a detection result after narrowing of the comparative example. Fig. 6C shows a detection result of the embodiment.

[Fig. 7] Fig. 7A shows a detection result (leaf detection result) before narrowing of the comparative example, and Fig. 7B shows a detection result after narrowing of the comparative example. Fig. 7C shows a detection result of the embodiment.

MODE FOR CARRYING OUT INVENTION

<Application example>

An application example of the present invention will be described.

A device (system) that detects (extracts) a leaf from a captured image and identifies a state of the detected leaf has been proposed. In such a device, when a leaf (for example, a leaf that looks elongated, a leaf that looks small, a leaf that is partially hidden by another leaf, a blurred leaf that is out of focus, a dark leaf, or the like.) that is not suitable for identification of a leaf state (state of a leaf) is detected, an incorrect identification result is obtained for the leaf, and an overall identification accuracy decreases. Then, in a case where the overall identification accuracy is low, work (labor) such as confirmation of the identification result by the agricultural expert (a person having specialized knowledge in agriculture) is required.

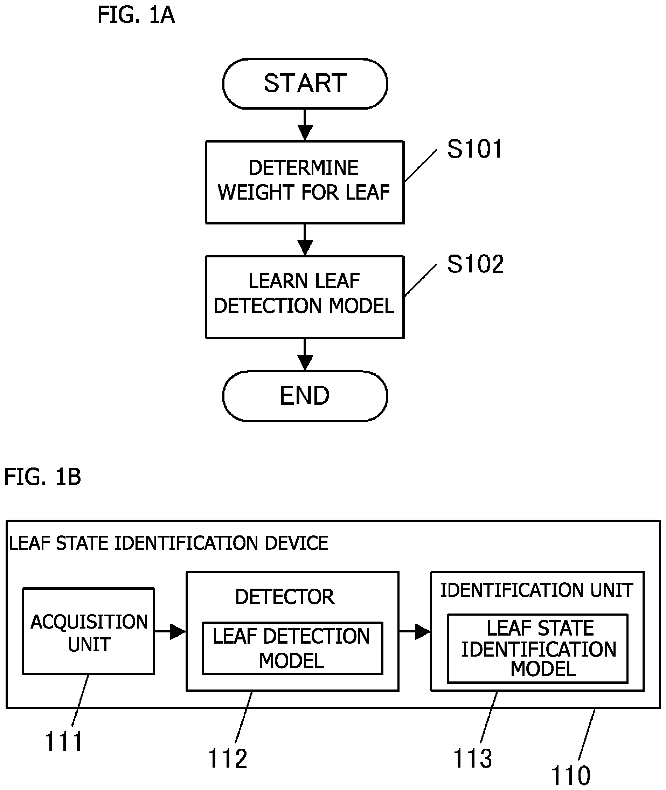

Fig. 1A is a flowchart illustrating an example of a learning method to which the present invention is applied. In step S101, a weight is determined for a leaf included in a captured image. In step S102, learning of a leaf detection model for detecting the leaf from the captured image is performed based on the weight determined in step S101 so that a leaf having a large weight is more easily detected than a leaf having a small weight. Step S101 is an example of a weight determination step, and step S102 is an example of a first learning step. The captured image may be or need not be a wide area image having a wide angle of view.

According to the above-described method, a weight is determined for the leaf, and the learning of the leaf detection model is performed such that the leaf having the large weight is more easily detected than the leaf having the small weight. In this way, a leaf can be suitably detected, and eventually, post-process such as identification of the leaf state can be performed with high accuracy. For example, when a large weight is determined for a leaf suitable for the post-process and a small weight is determined (or no weight is determined) for a leaf not suitable for the post-process, the leaf suitable for the post-process is more easily detected than the leaf not suitable for the post-process.

In step S101, a weight based on knowledge about agriculture may be determined. For example, in step S101, a weight based on knowledge obtained from at least one of a visual line of an agricultural expert and experience regarding agriculture may be determined. In this way, the large weight can be determined for the leave suitable for the post-process, and the small weight can be determined (or no weight can be determined) for the leave not suitable for the post-process. Information for the visual line may be acquired using an existing visual line detection technique.

Fig. 1B is a block diagram illustrating a configuration example of a leaf state identification device 110 to which the present invention is applied. The leaf state identification device 110 includes an acquisition unit 111, a detector 112, and an identification unit 113. The acquisition unit 111 acquires a captured image. The detector 112 detects a leaf from the captured image acquired by the acquisition unit 111 by using the leaf detection model learned by the learning method described above. The identification unit 113 identifies a state of the leaf detected by the detector 112 by using a leaf state identification model for identifying the state of the leaf. The acquisition unit 111 is an example of an acquisition section, the detector 112 is an example of a detection section, and the identification unit 113 is an example of an identification section. According to this configuration, the leaf is detected using the leaf detection model learned by the learning method described above, and thus the leaf state can be identified with high accuracy.

<Embodiment>

An embodiment of the present invention will be described.

(Configuration)

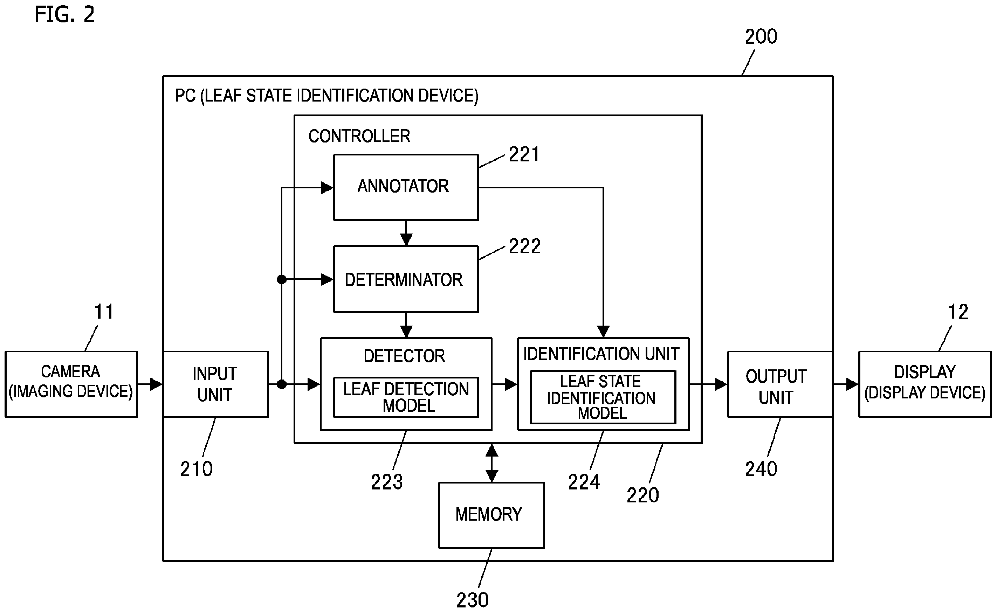

Fig. 2 is a block diagram illustrating a configuration example of a leaf state identification system according to the embodiment. The leaf state identification system includes a camera 11 (imaging device), a PC 200 (personal computer; a leaf state identification device) and a display 12 (display device). The camera 11 and the PC 200 are connected to each other by wire or wirelessly, and the PC 200 and the display 12 are connected to each other by wire or wirelessly. The camera 11 captures an image of a field or the like, and outputs the captured image thereof to the PC 200. The PC 200 detects a leaf from the captured image of the camera 11 and identifies a state of the detected leaf. Then, the PC 200 displays an identification result and the like on the display 12. The display 12 displays various images and information.

Note that the camera 11 may be or need not be fixed. A positional relationship among the camera 11, the PC 200, and the display 12 is not particularly limited. For example, the camera 11, the PC 200, and the display 12 may be or need not be installed in the same room (for example, plastic house).

In the embodiment, it is assumed that the camera 11 and the display 12 are separate devices from the PC 200, but at least one of the camera 11 and the display 12 may be a part of the PC 200. The PC 200 (leaf state identification device) may be a computer on a cloud. At least some of the functions of the camera 11, the PC 200, and the display 12 may be achieved by various terminals such as a smartphone and a tablet terminal.

The PC 200 includes an input unit 210, a controller 220, a memory 230, and an output unit 240.

The input unit 210 acquires the captured image from the camera 11. For example, the input unit 210 is an input terminal. The input unit 210 is an example of the acquisition section.

The controller 220 includes a central processing unit (CPU), a random access memory (RAM), a read only memory (ROM), and the like, and carries out control of each constituent element, various information processing, and the like. In the embodiment, the controller 220 detects a leaf from the captured image of the camera 11 (captured image acquired by the input unit 210) and identifies the state of the detected leaf.

The memory 230 stores programs executed by the controller 220, various data used by the controller 220, and the like. For example, the memory 230 is an auxiliary memory device such as a hard disk drive or a solid state drive.

The output unit 240 outputs the identification result of the controller 220 and the like to the display 12. As a result, the identification result and the like are displayed on the display 12. For example, the output unit 240 is an output terminal.

The controller 220 will be described in more detail. The controller 220 includes an annotator 221, a weight determinator 222, a detector 223, and an identification unit 224.

The annotator 221 performs annotation on the captured image of the camera 11. The weight determinator 222 determines a weight for a leaf included in the captured image of the camera 11. The detector 223 detects the leaf from the captured image of the camera 11 by using the leaf detection model. The identification unit 113 identifies a state of the leaf detected by the detector 112 by using the leaf state identification model. Details of these processes will be described later. The detector 112 is an example of the detection section and the identification unit 113 is an example of the identification section.

(Process flow of learning phase)

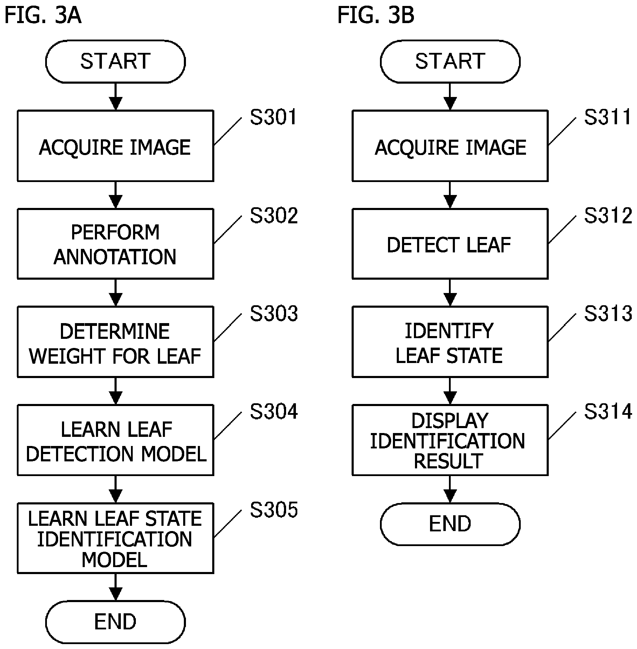

Fig. 3A is a flowchart illustrating a process flow example of the PC 200 in the learning phase. In the learning phase, learning of the leaf detection model is performed. In the embodiment, it is assumed that learning of the leaf state identification model is also performed.

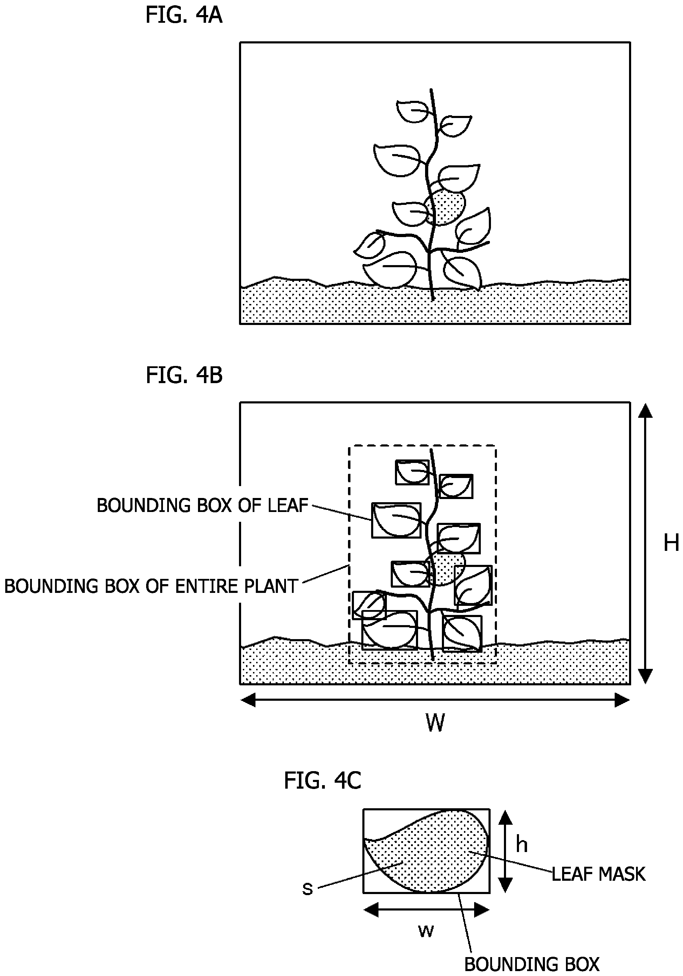

First, the input unit 210 acquires a captured image for learning (step S301). The captured image for learning may be or need not be a captured image of the camera 11. Fig. 4A shows an example of the captured image for learning. Although one plant appears in the captured image of Fig. 4A, a large number of plants may appear in the captured image.

Next, the annotator 221 performs annotation on the captured image acquired in step S301 (step S302). The annotation is a process of setting a true value (correct answer) in learning, and the true value is designated based on information designated (input) by an operator.

For example, the operator designates a contour of the leaf appearing in the captured image. In response to the designation of the contour, the annotator 221 sets a leaf mask in a region surrounded by the contour. Then, as illustrated in Fig. 4B, the annotator 221 automatically sets a bounding box that is a rectangular frame surrounding the leaf mask (leaf). For example, the annotator 221 sets, as the bounding box, a rectangular frame circumscribing the leaf mask (leaf).

Note that it is preferable that the operator selects only the leaf suitable for the post-process (identification of the leaf state in the embodiment) and designates the contour. However, it is difficult for a person other than an agricultural expert to determine whether the leaf is suitable for the post-process, and the operator who designates the contour is not necessarily the agricultural expert. Thus, in the annotation, the leaf mask or the bounding box of the leaf not suitable for the post-process may be set.

In the embodiment, as the identification of the leaf state, it is assumed that an identification whether the leaf is affected by diseases and insect pests (whether the leaf is healthy) is performed. Thus, the operator inputs information on whether the leaf is affected by the diseases and insect pests, and the annotator 221 sets the information. It is assumed that information on whether the leaf is affected by the diseases and insect pests is input by the agricultural expert. Note that in the identification of the leaf state, a type of a disease, a type of an insect pest, and the like may also be identified.

The description returns to Fig. 3A. After step S302, the weight determinator 222 determines a weight for the leaf included in the captured image acquired in step S301 based on the information set in step S302 (step S303). In the embodiment, the weight determinator 222 determines the weight of the leaf based on at least one of a shape, a size, and a position of the leaf. Step S302 is an example of the weight determination step.

A leaf that looks elongated by being viewed obliquely or partially hidden by another leaf or the like is likely to be not suitable for the post-process such that the leaf state cannot be identified with high accuracy. Thus, the weight determinator 222 may determine a larger weight for the leaf as the shape of the bounding box of the leaf is closer to a square. For example, the weight determinator 222 determines a weight $\omega 1$ from a width w and a height h of the bounding box illustrated in Fig. 4C by using the following Equations 1-1 and 1-2.

When w/h ≤ 1:

$$\omega1 = w/h \cdots \text{(Equation 1-1)}$$

When w/h > 1:

$$\omega1 = h/w \cdots \text{(Equation 1-2)}$$

A leaf that is undeveloped or partially hidden by another leaf or the like is likely to be not suitable for the post-process such that the leaf state cannot be identified with high accuracy. Thus, the weight determinator 222 may determine a larger weight for the leaf as the size of the leaf is larger. For example, the weight determinator 222 determines a weight $\omega2$ from a width W (the number of pixels in the horizontal direction) and a height H (the number of pixels in the vertical direction) of the captured image shown in Fig. 4B and the number of pixels s of the leaf mask shown in Fig. 4C by using the following Equation 2. W × H is the total number of pixels of the captured image.

$$\omega2 = s/(W \times H) \cdots \text{(Equation 2)}$$

The weight determinator 222 may determine the weight $\omega2$ by using the following Equations 2-1 to 2-3. Threshold values Th1 and Th2 are not particularly limited, but for example, in a case of W = 1200 and H = 1000, Th1 = 5000 and Th2 = 10,000 may be set. Note that the number of stages of the weight $\omega2$ may be more or less than three stages.

When s ≤ Th1:

$$\omega2 = 0.1 \cdots \text{(Equation 2-1)}$$

When Th1 < s ≤ Th2:

$$\omega2 = 0.5 \cdots \text{(Equation 2-2)}$$

When Th2< s:

$$\omega2 = 0.9 \cdots \text{(Equation 2-3)}$$

Since the closer to the ground, the higher the humidity, mold disease is more likely to occur in the leaf closer to the ground than in the leaf farther from the ground. Thus, the weight determinator 222 may determine a larger weight for the leaf as the leaf is closer to the ground. For example, in a case where the captured image is an image in which a plant is imaged from the side, the weight determinator 222 determines a weight $\omega3$ from a vertical position c_y (position in the vertical direction) of the center of the bounding box by using Equation 3-1 to 3-3. Threshold values Th3 and Th4 are not particularly limited, but for example, the threshold value Th3 corresponds to a vertical position where a vertical distance (distance in the vertical direction) from a lower end of the captured image is H/3, and the threshold value Th4 corresponds to a vertical position where a vertical distance from the lower end of the captured image is (2/3) × H. Here, it is assumed that a value (coordinate value) of the vertical position increases from a lower end to an upper end of the captured image. Note that the number of stages of the weight $\omega3$ may be more or less than three stages.

When c_y ≤ Th3:

$$\omega3 = 0.9 \cdots \text{(Equation 3-1)}$$

When Th3<c_y ≤ Th4:

$$\omega3 = 0.5 \cdots \text{(Equation 3-2)}$$

When Th4<c_ y:

$$\omega 3 = 0.1 \cdots \text{(Equation 3-3)}$$

In a case where the captured image is an image obtained by capturing a field in a bird's eye view, a leaf close to the ground may be positioned on an upper portion of the captured image. In such a case, a bounding box of the entire plant is set as illustrated in Fig. 4B, and a vertical distance from the lower end of the bounding box of the entire plant, instead of a vertical distance from the lower end of the captured image, may be regarded as the distance from the ground.

The weight determinator 222 may determine any one of the weights $\omega 1$ to $\omega 3$ described above, or may determine a final weight $\omega$ by combining two or three of the weights $\omega 1$ to $\omega 3$. For example, the weight determinator 222 may determine $\omega 1 \times \omega 2$, $\omega 1 \times \omega 3$, $\omega 2 \times \omega 3$, or $\omega 1 \times \omega 2 \times \omega 3$ as the final weight $\omega$. In addition, the weight determinator 222 may determine the weight $\omega$ only for a leaf satisfying a predetermined condition ($\omega = 0$ may be determined for a leaf not satisfying the predetermined condition). The predetermined condition may include a condition of 0.75 < w/h < 1.3. When W = 1200 and H = 1000, the predetermined condition may include a condition of s > 10,000.

Note that the determining method of the weight is not limited to the above method. For example, since young leaves (upper leaves) are more affected by insect pests, the weight determinator 222 may determine a larger weight for a leaf as the leaf is farther from the ground. The weight determinator 222 may increase the weight of a leaf with appropriate exposure (appropriate brightness) or increase the weight of a clear leaf based on a luminance value or definition of the image of the leaf.

The description returns to Fig. 3A. After step S303, the controller 220 performs learning of the leaf detection model included in the detector 223 based on the weight determined in step S303 so that the leaf having the large weight is more easily detected than the leaf having the small weight (step S304). Step S304 is an example of the first learning step. By performing learning of the leaf detection model so that the leaf having the large weight is more easily detected than the leaf having the small weight, the leaf can be suitably detected, and eventually, the post-process such as identification of the leaf state can be performed with high accuracy.

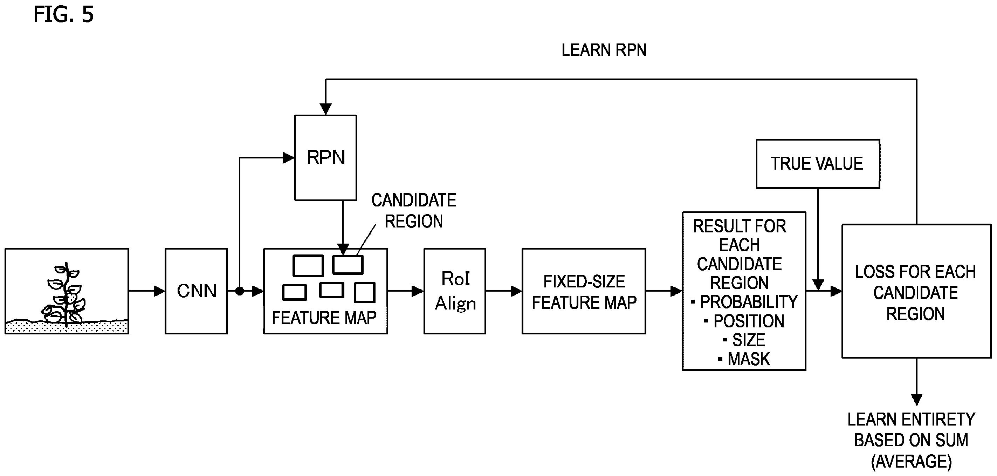

Various methods such as Mask R-CNN and Faster R-CNN can be used for the leaf detection model. In the embodiment, as illustrated in Fig. 5, it is assumed that the leaf detection model is an inference model (learning model) using Mask R-CNN. Mask R-CNN is a known method, and thus an outline thereof will be described below.

In the leaf detection model (Mask R-CNN), first, a feature amount is extracted from the captured image by a convolutional neural network (CNN), and a feature map is generated. Next, a candidate region that is a candidate for a region of a leaf (bounding box) is detected from the feature map by RPN. Then, a fixed-size feature map is obtained by RoI Align, and an inference result (a probability (correct answer probability) that the candidate region is the region of the leaf, a position of the candidate region, a size of the candidate region, a candidate of a leaf mask, and the like) for each candidate region is obtained through a process of an entire connected layer (not illustrated) or the like. After learning the leaf detection model, the detector 223 detects the candidate region whose correct answer probability is a predetermined threshold value or more as the bounding box of the leaf.

At the time of learning the leaf detection model, the controller 220 calculates a loss L by comparing the inference result with the true value (correct answer) for each candidate region. The loss L is calculated, for example, using the following Equation 4 (loss function). A loss Lcls is a classification loss of the bounding box, and becomes small when the candidate region matches a correct bounding box. A loss Lloc is a regression loss of the bounding box, and is smaller as the candidate region is closer to the correct bounding box. A loss Lmask is a matching loss of the leaf mask, and is smaller as the candidate of the leaf mask is closer to the correct leaf mask. Coefficients $f(\omega)$ and $g(\omega)$ are coefficients depending on the weight $\omega$ determined by the weight determinator 222, and for example, $f(\omega) = g(\omega) = e^{-\omega}$. In the embodiment, the weight determinator 222 determines the weight of the leaf based on at least one of the shape, size, and position of the leaf. Since losses related to the shape, size, and position of the leaf are the loss Lloc and the loss Lmask, the loss Lloc and the loss Lmask are multiplied by the coefficients $f(\omega)$ and $g(\omega)$, respectively.

$$L = \text{Lcls} + \text{Lloc} \times f(\omega) + \text{Lmask} \times g(\omega) \cdots \text{(Equation 4)}$$

Then, the controller 220 updates the RPN based on the loss L for each candidate region. The coefficients $f(\omega)$ and $g(\omega)$ are smaller as the weight $\omega$ is larger. Thus, a value of the loss function (L = Lcls + Lloc + Lmask) not considering the weight $\omega$ is reduced with a larger reduction amount as the weight $\omega$ is larger. By updating the RPN based on the loss L thus reduced, the allowable range of the leaf is adjusted such that the allowable range based on the leaf having the large weight $\omega$ is wide and the allowable range based on the leaf having the small weight $\omega$ is narrow. As a result, the candidate region of the leaf having the large weight $\omega$ (a leaf included in the allowable range based on the leaf having the large weight $\omega$) is more easily detected than the candidate region of the leaf having the small weight $\omega$ (a leaf included in the allowable range based on the leaf having the small weight $\omega$). Further, the controller 220 updates the entire leaf detection model based on the sum (average) of the losses L for candidate regions, respectively.

Note that, although the example of reducing the candidate region of the leaf having the small weight ω has been described, the leaf having the large weight ω may be more easily detected than the leaf having the small weight ω by another method. For example, learning of the leaf detection model may be performed so as to reduce the correct answer probability of the candidate region of the leaf having the small weight ω.

The description returns to Fig. 3A. After step S304, the controller 220 performs learning of the leaf state identification model included in the identification unit 224 by using the detection result of the detector 223 including the leaf detection model learned in step S304 (step S305). Step S305 is an example of the second learning step. By using the detection result of the detector 223 including the leaf detection model which is learned, the leaf state identification model that can identify a leaf with high accuracy can be obtained. Various methods can also be used for the leaf state identification model.

(Process flow of inference phase)

Fig. 3B is a flowchart illustrating a process flow example of the PC 200 in the inference phase after the learning phase. First, the input unit 210 acquires a captured image from the camera 11 (step S311). Next, the detector 223 detects a leaf from the captured image acquired in step S311 by using the leaf detection model which is learned (step S312). Next, the identification unit 113 identifies the state of the leaf detected in step S312 by using the leaf state identification model which is learned (step S313). Next, the output unit 240 outputs and displays the identification result of step S313 to the display 12 (step S314).

(Effects)

Effects of the embodiment will be described. In the embodiment, a weight is determined for the leaf, and the learning of the leaf detection model is performed such that the leaf having the large weight is more easily detected than the leaf having the small weight. As another method (comparative example), a method of narrowing the leaf detection result with a predetermined threshold value is considered. However, with such a method, a detection result (leaf detection result) as suitable as the method of the embodiment cannot be obtained.

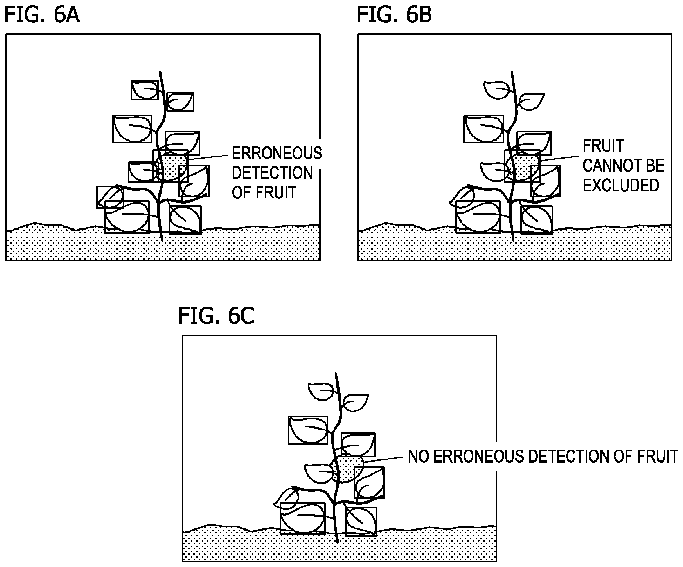

Fig. 6A and Fig.6B show detection results of the comparative example. Fig. 6A shows the detection result before narrowing. Since, in learning, a weight is not considered, all leaves are detected. Further, a fruit is erroneously detected. Fig. 6B shows a result of narrowing with a size threshold value in order to remove small leaves. In Fig. 6B, the small leaves are excluded from the detection result, but the fruit is not excluded because it is large.

Fig. 6C shows a detection result of the embodiment. By considering the weight in learning (learning was performed by increasing the weight of the leaf that well represents the characteristics of the leaf), neither the small leaf nor the fruit is detected, and only the large leaf suitable for the post-process can be detected.

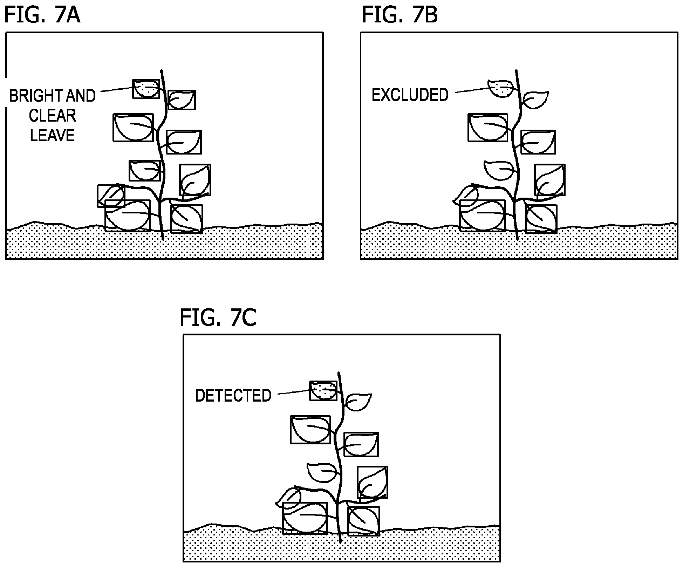

Fig. 7A and Fig. 7B show detection results of the comparative example. Fig. 7A shows the detection result before narrowing. Since, in learning, a weight is not considered, all leaves are detected. A bright and clear leave has also been detected. Such a leaf is likely to be a leaf suitable for the post-processing (for example, a leaf whose leaf state can be identified with high accuracy) even when it is small. Fig. 7B shows a result of narrowing with a size threshold value in order to remove small leaves. In Fig. 7B, the bright and clear leave that should be left as the leave suitable for the post-process is excluded due to its small size.

Fig. 7C shows a detection result of the embodiment. Although it is difficult to detect the small leaf by considering the weight in learning, the bright and clear leaf can be detected even when it is small because it well represents the characteristics of the leaf.

(Summary)

As described above, according to the embodiment, a weight is determined for the leaf, and the learning of the leaf detection model is performed such that the leaf having the large weight is more easily detected than the leaf having the small weight. In this way, a leaf can be suitably detected, and eventually, post-process such as identification of the leaf state can be performed with high accuracy.

<Others>

The above embodiments merely describe, as examples, the configuration examples of the present invention. The present invention is not limited to the specific forms described above, and various modifications can be made within the scope of the technical idea.

**EP 4 471 705 A1**

<Supplementary note 1 >

A learning method includes

a weight determination step (S101 and S303) of determining a weight for a leaf included in a captured image; and a first learning step (S102 and S304) of performing learning of a leaf detection model for detecting a leaf from the captured image based on the weight determined in the weight determination step such that a leaf having a large weight is more easily detected than a leaf having a small weight.

<Supplementary note 2>

A leaf state identification device (110 and 200) includes

an acquisition section (111 and 210) configured to acquire a captured image,
a detection section (112 and 223) configured to detect a leaf from the captured image acquired by the acquisition section by using the leaf detection model learned by the learning method according to any one of claims 1 to 9, and an identification section (113 and 224) configured to identify a state of the leaf detected by the detection section by using a leaf state identification model configured to identify a state of a leaf.

DESCRIPTION OF SYMBOLS

110: leaf state identification device 111: acquisition unit 112: detector
113: identification unit
200: PC (information process device)
210: input unit 220: controller 230: memory 240: output unit
221: annotator 222: weight determinator 223: detector 224: identification unit
11: camera 12: display

## Claims

1. A learning method comprising:

   a weight determination step of determining a weight for a leaf included in a captured image; and a first learning step of performing learning of a leaf detection model configured to detect a leaf from the captured image to cause a leaf having a large weight to be more easily detected than a leaf having a small weight based on the weight determined in the weight determination step.

2. The learning method according to claim 1, wherein in the weight determination step, a weight based on knowledge about agriculture is determined.

3. The learning method according to claim 2, wherein in the weight determination step, a weight based on knowledge obtained from at least one of a visual line of an agricultural expert and experience regarding agriculture is determined.

4. The learning method according to claim 3, wherein in the weight determination step, the weight of the leaf is determined based on at least one of a shape, a size, and a position of the leaf.

5. The learning method according to claim 4, wherein in the weight determination step, a larger weight for the leaf is determined as a shape of a bounding box of the leaf is closer to a square.

6. The learning method according to claim 4 or 5, wherein in the weight determination step, a larger weight is determined for a leaf as a size of the leaf is larger.

7. The learning method according to any one of claims 4 to 6, wherein in the weight determination step, a larger weight is determined for a leaf as the leaf is closer to the ground.

8. The learning method according to any one of claims 4 to 6, wherein in the weight determination step, a larger weight is determined for a leaf as the leaf is farther from the ground.

9. The learning method according to any one of claims 1 to 8, wherein the leaf detection model is an inference model using Mask R-CNN or Faster R-CNN.

10. The learning method according to claim 9, wherein in the first learning step, a value of a loss function is reduced with a larger reduction amount as the weight is larger.

11. The learning method according to any one of claims 1 to 10, further comprising a second learning step of performing learning of a leaf state identification model configured to identify a state of a leaf by using a detection result of the leaf detection model learned in the first learning step.

12. The learning method according to claim 11, wherein the leaf state identification model identifies whether a leaf is affected by diseases and insect pests.

13. A leaf state identification device comprising:

   an acquisition section configured to acquire a captured image;
   a detection section configured to detect a leaf from the captured image acquired by the acquisition section by using the leaf detection model learned by the learning method according to any one of claims 1 to 12; and
   an identification section configured to identify a state of the leaf detected by the detection section by using a leaf state identification model configured to identify a state of a leaf.

14. A program configured to cause a computer to perform the steps of the learning method according to any one of claims 1 to 12.

FIG. 1A

```
                    ┌─────────────┐
                    │    START    │
                    └──────┬──────┘
                           │
                           ▼
              ┌───────────────────────┐
              │      DETERMINE        │        S101
              │   WEIGHT FOR LEAF     │
              └───────────┬───────────┘
                          │
                          ▼
              ┌───────────────────────┐
              │     LEARN LEAF        │        S102
              │  DETECTION MODEL      │
              └───────────┬───────────┘
                          │
                          ▼
                    ┌─────────────┐
                    │     END     │
                    └─────────────┘
```

FIG. 1B

**FIG. 2**

EP 4 471 705 A1

## FIG. 3A

START

ACQUIRE IMAGE — S301

PERFORM ANNOTATION — S302

DETERMINE WEIGHT FOR LEAF — S303

LEARN LEAF DETECTION MODEL — S304

LEARN LEAF STATE IDENTIFICATION MODEL — S305

END

## FIG. 3B

START

ACQUIRE IMAGE — S311

DETECT LEAF — S312

IDENTIFY LEAF STATE — S313

DISPLAY IDENTIFICATION RESULT — S314

END

FIG. 4A

FIG. 4B

BOUNDING BOX OF LEAF

BOUNDING BOX OF ENTIRE PLANT

H

W

FIG. 4C

s

h

LEAF MASK

w

BOUNDING BOX

FIG. 5

CNN

FEATURE MAP

CANDIDATE REGION

RPN

LEARN RPN

RoI Align

FIXED-SIZE FEATURE MAP

RESULT FOR EACH CANDIDATE REGION
- PROBABILITY
- POSITION
- SIZE
- MASK

TRUE VALUE

LOSS FOR EACH CANDIDATE REGION

LEARN ENTIRETY BASED ON SUM (AVERAGE)

FIG. 6A

ERRONEOUS
DETECTION
OF FRUIT

FIG. 6B

FRUIT
CANNOT BE
EXCLUDED

FIG. 6C

NO ERRONEOUS DETECTION OF FRUIT

FIG. 7A

BRIGHT AND
CLEAR
LEAVE

FIG. 7B

EXCLUDED

FIG. 7C

DETECTED

## INTERNATIONAL SEARCH REPORT

| International application No. |
| --- |
| **PCT/JP2022/011125** |

### A. CLASSIFICATION OF SUBJECT MATTER

*G06Q 50/02*(2012.01)i; *A01G 7/00*(2006.01)i
FI:   A01G7/00 603; G06Q50/02

According to International Patent Classification (IPC) or to both national classification and IPC

### B. FIELDS SEARCHED

Minimum documentation searched (classification system followed by classification symbols)

G06Q50/02; A01G7/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| A | JP 2013-5726 A (NIKON CORP) 10 January 2013 (2013-01-10)<br>paragraphs [0076]-[0080] | 1-14 |
| A | JP 2021-112136 A (YOKOGAWA ELECTRIC CORP) 05 August 2021 (2021-08-05)<br>claims | 1-14 |
| A | WO 2018/047726 A1 (BOSCH CORP) 15 March 2018 (2018-03-15)<br>claims | 1-14 |
| A | JP 2021-39757 A (NAT AGRICULTURE & FOOD RES ORG) 11 March 2021 (2021-03-11)<br>claims | 1-14 |
| A | JP 2020-141590 A (NEC CORP) 10 September 2020 (2020-09-10)<br>claims | 1-14 |

☐ Further documents are listed in the continuation of Box C.   ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
| --- | --- |
| **24 May 2022** | **31 May 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/011125**

| Patent document cited in search report | | | Publication date (day/month/year) | Patent family member(s) | | | Publication date (day/month/year) |
|---|---|---|---|---|---|---|---|
| JP | 2013-5726 | A | 10 January 2013 | (Family: none) | | | |
| JP | 2021-112136 | A | 05 August 2021 | (Family: none) | | | |
| WO | 2018/047726 | A1 | 15 March 2018 | EP claims | 3550498 | A1 | |
| | | | | CN | 109643431 | A | |
| | | | | KR | 10-2019-0046841 | A | |
| JP | 2021-39757 | A | 11 March 2021 | (Family: none) | | | |
| JP | 2020-141590 | A | 10 September 2020 | US claims | 2020/0285941 | A1 | |

Form PCT/ISA/210 (patent family annex) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Proceedings of the Graduate School of Science and Technology. Hosei University, 31 March 2017, vol. 58, 1-4